# EUROPEAN PATENT SPECIFICATION

(11) **EP 1 301 165 B1**
(45) Date of publication and mention of the grant of the patent: **31.01.2007**
(21) Application number: 01947655.5
(22) Date of filing: 06.07.2001
(51) Int. Cl.: A61K 8/72, A61Q 19/00

(54) **USE OF AN ALLOMELANIN FOR THE TREATMENT OF INFLAMMATION OR IRRITANCY CAUSED BY DHA AND/OR A SURFACTANT**
VERWENDUNG VON ALLOMELANINEN FÜR BEHANDLUNG VON ENTZÜNDUNGEN ODER INRRITATIONEN VERURSACHT DURCH DHA UND/ODER EIN TENSID
UTILISATION D'UNE ALLOMELANINE POUR TRAITER L'INFLAMMATION OU L'IRRITATION DUE À LA DHA ET/OU UN TENSIOACTIF

(30) Priority: 21.07.2000 GB 0018019; 04.05.2001 GB 0111069
(43) Date of publication of application: 16.04.2003
(73) Proprietor: Croda Chemicals International Limited, Goole, East Yorkshire DN14 9AA (GB)
(72) Inventor: CHEETHAM, Peter, Samuel, James, Tunbridge Wells, Kent TN2 5HW (GB); TAYLOR, Charles, Jefferson, Emsworth, Hampshire PO10 7PU (GB)
(74) Representative: Coates, Ian Harold
(86) International application number: PCT/GB2001/003061
(87) International publication number: WO 2002/007696

(56) References cited:
- WO-A-01/18125
- WO-A-95/09629
- WO-A-97/20944
- DATABASE WPI Week 199344 Derwent Publications Ltd., London, GB; AN 1993-348319 XP002179879 & JP 05 255037 A (EISAI CO)
- DATABASE CAPLUS [Online] CHEMICAL ABSTRACTS SERVICE, COLUMBUS, OHIO, US; retrieved from STN Database accession no. 1997:424793 XP002179878 & JP 09 151130 A (YAKURIGAKU CHUO KKK) 10 June 1997 (1997-06-10)

## Description

### Technical Field

The present invention relates to compositions containing melanin pigments, particularly cosmetic compositions.

### Background Art

Our earlier application WO97/20944 discloses a method of producing a melanin by oxidising a phenolic compound using an oxidoreductase enzyme. The phenolic compound may be of formula (I): 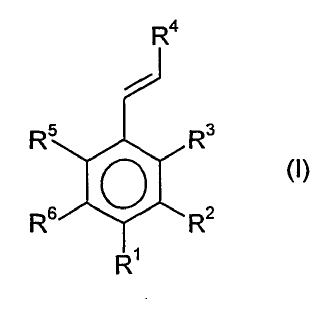 where R¹ is H or OH, R² is H, OH or OCH₃, R³ is H or OH (provided that at least one of R¹ and R³ is OH);
R⁴ is selected from H, R, -COOX and R⁷-COOX, wherein R is an optionally substituted saturated or unsaturated alkyl group having from 1 to 12 carbon atoms R⁷ is an optionally substituted saturated or unsaturated alkylene group having from 1 to 12 carbon atoms and X is selected from H and aliphatic and aromatic ester forming groups; and
R⁵ and R⁶ are each independently selected from H, OH, NH₂, OCH₃, CH₃, SH, NHCO₂, NHCH₃ COOH and saturated or unsaturated alkyl groups having up to 8 carbon atoms.

Suitable enzymes include tyrosinases, laccases and peroxidases.

Melanins derived from caffeic acid (I, R₁=R₂=OH; R₃=R₅=R₆=H; R₄=CO₂H) and ferulic acid (I, R₁=OH, R₂=OMe, R₃=R₅=R₆=H, R₄=CO₂H) and their derivatives are of the class known as allomelanins which do not contain sulphur or nitrogen. They are relatively light in colour, whereas conventional melanins are black (unless bleached, which is disadvantageous in various ways).

Our application WO95/33706 discloses the preparation of caffeic acid by enzymatic treatment of materials derived from plants, e.g. sunflowers.

### Disclosure of Invention

The present invention is primarily concerned with allomelanins, particularly those derived from caffeic and ferulic acids and their derivatives, and especially those derived from caffeic acid and derivatives ("caffeic melanin materials'').

We have now found that our allomelanins, particularly caffeic melanin materials, can be extremely soluble in water, e.g. 150g/l at ambient temperatures. In contrast, conventional melanins are virtually insoluble. Our melanin derived from caffeic acid has a solubility of about 220g/l. Since caffeic acid has a solubility of below 1g/l, and polymerisation generally leads to a reduction in solubility, this is quite remarkable.

WO97/20944 states that it provides melanins which are "generally more soluble in other ingredients conventionally used in the preparation of formulations for cosmetic and other applications than melanins made industrially in the prior art". This is not explained further or exemplified. The only apparent evidence of the solubility of the melanin is that UV spectra are determined for 0.005% w/v aqueous solutions. Clearly there is nothing to suggest materials whose solubilities are orders of magnitude greater than those of conventional melanins. Our discovery of this has made possible the production of cosmetics making new uses of melanins, depending on their high solubilities for the preparation of the cosmetic formulations and/or their modes of action. Thus solutions of our melanins can be effective as antioxidants and UV (A and B) absorbers. They can be formulated so that they adhere to hair and skin, and can serve as colorants of hair or skin.

We have also found that our allomelanins can lead to compositions of greater stability than can be achieved with conventional melanins. For example a composition containing dihydroxyacetone and a melanin rapidly forms a precipitate if the melanin is conventional, whereas use of our allomelanins gives a composition that is stable for many weeks.

Our allomelanins have good UV-absorbing properties and antioxidant activity. These properties together with the moderate colourations and high solubility make them suitable for a wide range of cosmetic compositions.

Tests on the UV absorbing properties of our caffeic acid derived melanin showed that a 0.5% w/w formulation gives a cream with a Sun Protection Factor of ca 2 and a star rating of 3, which is categorised as excellent. The former is chiefly a measure of absorption of UV B radiation and is a good estimate of protection from sunburn. The latter is an estimate of UV A absorption and the anti-ageing efficacy of the cream.

It is provided a cosmetic composition containing an allomelanin having a water solubility of at least 20g/l and/or being substantially stable in the presence of dihydroxyacetone such that substantially no precipitate is formed when a composition containing the allomelanin and dihydroxyacetone is stored at ambient temperature for 2 weeks. Preferably the composition is stable for in excess of a month, e.g. for 3 months or more.

The water solubility is preferably at least 50g/l, more preferably at least 100g/l. The composition may comprise an aqueous solution containing at least 20g/l of allomelanin and/or may be produced using such a solution.

A preferred type of composition contains both allomelanin and dihydroxyacetone.

The allomelanin is preferably one produced by the enzymatic oxidation of a phenol or mixture of phenols of formula (I) above, wherein R¹ is OH; R² and R⁶ are independently selected from H, OH and OMe; R³ and R⁵ are H; and R⁴ is CO₂X, particularly caffeic acid and/or ferulic acid and/or an ester or mixture of esters thereof. When -CO₂X is an ester group, X is preferably C₁₋₆ alkyl e.g. methyl or ethyl. Peroxidase enzymes are preferred, e.g. horseradish, soybean and microbial peroxidases.

We have also found that our allomelanins have anti-inflammatory or anti-irritancy properties. Thus they can be incorporated in compositions, particularly cosmetic compositions, to reduce potential irritancy associated with other components present in the composition or other agents that may affect the skin, such as components of other compositions that may be applied to the skin. For example, some people are prone to irritation by dihydroxyacetone and/or surfactants.

Thus the invention provides the use of an allomelanin for reducing inflammation or irritation or potential irritation, caused by dihydroxyacetone and/or surfactant, generally for topical application.

Some preferred types of embodiment will now be outlined.
1. Use of an allomelanin in a cosmetic composition as an anti-inflammatory or anti-irritancy agent, e.g. for countering potential effects of one or more other components of the composition, said allomelanin being a material produced by enzymatic oxidation of at least one phenol of formula II: 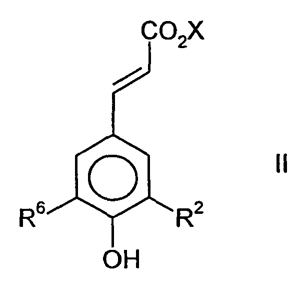 where R² and R⁶ are independently selected from H, OH and OMe and X is H or a C₁₋₆ alkyl group. Preferably the allomelanin has a water solubility of at least 50g/l at room temperature. Preferably the amount of the melanin is sufficient to reduce substantially the skin irritant effect of the composition.
2. Use according to (1) as a colorant for hair or skin.
3. Use according to (1) or (2) wherein the cosmetic composition also contains dihydroxyacetone.

Cosmetic compositions include sunscreens, moisturisers, shampoos, conditioners, sunless tanning compositions (such as lotions and creams), fragrances, and lipsticks and lip balms.

In fragrance compositions the melanin can serve (a) to protect other ingredients from oxidation; (b) to act as a UV absorbent to protect against photochemical degradation; (c) to provide an attractive bronze colour; and (d) as an anti-irritant. Fragrance components commonly have irritant properties.

In lip balms and the like, in addition to properties mentioned elsewhere, the melanin can provide antiviral activity effective against the *Herpes simplex* virus responsible for cold sores. Since outbreaks of cold sores can be triggered by UV exposure (excessive sun), such lip balms can provide a double protection.

In a sunscreen, melanin serves as a UV absorbent, and also as an antioxidant (protecting the other ingredients) and as a colorant or pigment. It may reduce irritation.

Tinting compositions for the hair (shampoos, conditioners, lotions etc.) desirably incorporate our melanin and a hair penetration enhancer, e.g. ethoxydiglycol. The melanin can provide both pigmentation and anti-inflammatory action. Preferably the compositions are formulated to be used at a slightly basic pH, e.g. in the range pH 7-9 preferably around pH 8.0 (as compared with the acidic pH=s, 3.5-4.0, commonly used for conditioners). This opens up the cuticles on the hair shaft so as to facilitate deposition of melanin. A fixing compound, e.g. a quaternary ammonium compound, may be used to fix melanin onto the hair. A follow-up low pH conditioner may be used to close up the hair follicles again.

A sunless tanning composition may include dihydroxyacetone ('DHA') as a skin tanning agent. A typical composition employs, by weight: 0.05 - 0.5% melanin (preferably 0.1 - 0.3%) and 0.4 - 10% DHA (preferably 2 - 5%) and has a pH in the range 2 - 7 (preferably 4 - 6). It preferably also includes erythrulose. When erythrulose is used the skin is less dry and flaky after use of the DHA, leading to a better artificial tan in terms of depth, evenness (non-streakiness) and length of tan.

Tanning lotions and other skin creams may contain a film-forming material (e.g. Antarron V220) and/or a quaternary compound (e.g. Stepanquat ML) to assist in fixing melanin to the skin.

Compositions generally include additional components such as one or more vehicles. The particular choice of vehicle will depend on the method chosen for administration as well as on the site to which the composition is applied.

Vehicles can include water or substances such as liquid or solid emollients, solvents, humectants, thickeners and powders. Examples of each of these types of vehicle, which can be used singly or as mixtures of one or more vehicles, are as follows:
Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;
Propellants, such as trichlorofluoromethane, dichlorodifluoromethane, dichlorotetrafluroethane, monochlorodifluoromethane, trichlorotrifluoroethane, propane, butane, isobutane, dimethyl ether, carbon dioxide, nitrous oxide;
Solvents, such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;
Humectants, such as glycerin, sorbitol, sodium 2-pyrrolidone-5-carboxylate, soluble collagen, dibutyl phthalate and gelatin;
Powders, such as chalk, talc, fullers earth, kaolin, starch, gums, colloidal silica, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the composition, and can, in the absence of other cosmetic adjuncts, form the balance of the composition.

The composition can also comprise other materials which are conventionally useful in cosmetic or therapeutic products for topical application to skin and hair. Examples include surfactants, especially anionic, nonionic and amphoteric surfactants, polymers such as Polymer JR and Jaguar gums, preservatives, perfumes, moisturisers, antioxidants, etc.

The compositions can be formulated as liquids, for example as a lotion, shampoo, milk or cream for use in conjunction with an applicator such as a roll-ball applicator, or a spray device such as an aerosol can containing propellant, or a container fitted with a pump to dispense the liquid product.

Alternatively, the compositions be solid or semi-solid, for example sticks, creams or gels, for use in conjunction with a suitable applicator or simply a tube, bottle or lidded jar, or as a liquid-impregnated fabric, such as a tissue wipe.

### Modes for Carrying Out the Invention

Some embodiments of the invention will now be described in more detail by way of example.

### A) Preparation of allomelanin

Suitable allomelanins may be prepared from caffeic acid, optionally mixed with ferulic acid, as described in examples in WO97/20944. Examples 4 and 11 thereof are reproduced below as examples Ai and Aii.

### Ai) Production of a caffeic acid/ferulic acid melanin by horseradish peroxidase plus hydrogen peroxide.

Caffeic acid (500mg) and ferulic acid (500mg) were dissolved in ethanol (20ml) plus citrate buffer (100ml, 100mM, pH 5.5) in a 500ml Erlenmeyer flask. Horseradish peroxidase (500 units, Sigma) and hydrogen peroxide (5ml, 27.5%) were added and the solution was incubated for 2 hours at 30°C, shaken at 200 rpm. Utilisation of caffeic acid and ferulic acid was monitored by HPLC (C18 column, 2ml/min, water:acetonitrile (80:20) + acetic acid (1%), 290nm). The reaction was terminated by addition of concentrated hydrochloric acid (10ml) to the solution cooled on ice. A red/brown precipitate was collected by centrifugation (4000 rpm, 15 min). The red/brown paste was air dried. Yield: 126.3mg (12.63%).

### Aii) Production of a caffeic acid derived allomelanin by tyrosinase using evaporation.

Caffeic acid (5g) was dissolved in water (21) and adjusted to pH 7.0 using sodium hydroxide (5N). Tyrosinase (17mg, 4400 units/mg, Sigma) was added and the solution equally divided between two 51 Erlenmeyer flasks. Reaction was incubated overnight at 30°C and shaken at 200 rpm. Product was recovered by evaporation at reduced pressure using a rotary evaporator and oven dried at 50°C to yield a brown coloured powder. Yield: 5.5g (100%).

### Aiii)(a) Production of caffeic acid from sunflower meal

13kg non-pelleted sunflower meal was suspended in 37L water at room temperature and mixed by hand. The aqueous phase was monitored by HPLC for chlorogenic acid release until no further increase occurred.

The sunflower meal suspension was pressed to release the aqueous phase using a Vigo wine press (36L capacity). The solids obtained were washed with a volume of water equivalent to the initial volume minus the volume obtained from the first press. This suspension was pressed again, and the aqueous phases from both pressings combined.

The pH of the aqueous was adjusted to and maintained at pH 7.5 with 400gL⁻¹ NaOH, and heated to 40°C, with mixing in a 50L stainless steel vessel. 4mlL⁻¹ Biofeed Plus L enzyme (Novo Nordisk) was added, and the chlorogenic acid conversion to caffeic acid monitored until the caffeic acid concentration attained a plateau.

The aqueous pH was dropped to pH 3.0 using 750 gL⁻¹ phosphoric acid. The precipitate formed was removed by centrifugation using the Westfalia SC-6.

The clarified liquor was transferred to a flame proof stainless steel vessel, and overlaid with 10L of ethyl acetate. The two phases were stirred gently overnight to allow the extraction of the caffeic acid into the solvent as a two-phase system. The ethyl acetate layer was then decanted off.

20 batches of sunflower meal extraction were performed using the method described above, and the ethyl acetate layers obtained were pooled and concentrated by evaporation *in vacuo,* using a QVF distillation apparatus followed by a Buchi rotary evaporator.

The solids obtained were washed with a small volume of n-hexane, and the washed solids recovered by filtration through Whatman no. 1 filter paper under vacuum and then dried in a vacuum oven.

This gave 504g of sunflower meal extract containing 306g caffeic acid (therefore the extract is 61% w/w caffeic acid). The average yield per batch is therefore 25g of extract, containing 15g caffeic acid, and the overall yield for caffeic acid from sunflower meal using this process on this meal is 0.11% w/w.

### Aiii) (b) Production of melanin from extract containing caffeic acid

450g of the sunflower meal extract described (containing 273g caffeic acid) was dissolved in 45L of distilled water in a stainless steel vessel. The pH of this solution was adjusted to and held at pH 7.5 using 400gL⁻¹ NaOH. 2g of soybean peroxidase enzyme (Organic Technologies Ltd.) was added to this solution. The melanin production reaction was initiated by the addition, dropwise over a period of 30 minutes, of 211ml of a 27% w/v hydrogen peroxide solution.

After 2 hours, the reaction was cooled to 20°C and stirred overnight. The melanin solution generated was then concentrated by evaporation *in vacuo,* using the QVF distillation apparatus followed by a Buchi rotary evaporator. The concentrated melanin solution was transferred to drying dishes and taken to final dryness using a vacuum oven.

The dry melanin solid produced was scraped out of the drying dishes and ground into a fine dark brown powder using a pestle and mortar.

The final amount of melanin was 552g, containing 0.27% w/w caffeic acid.

Caffeic acid-derived melanin as produced in preparative example (Aii) was used in the following formulations.

### B) Formulations

Note: the components of each "stage" are mixed together and then the "stages" are blended.

### B (i) Melanin-Containing Sunscreen

| **Material** | **Grams** |
|---|---|
| **Stage A** | |
| Amphisol K | 5.00 |
| Parsol MCX | 37.50 |
| Lexemul 561 | 22.500 |
| Propyl Paraben | 1.000 |
| DL alpha-tocopheryl acetate | 0.500 |
| Ceteraryl alcohol | 15.000 |
| Light mineral oil | 30.000 |
| Silicone 593 | 10.000 |
| Parsol 1789 | 12.50 |

| **Stage B** | |
|---|---|
| Na₂EDTA | 0.25 |
| Water, pure | To 500g |
| Propylene glycol mono | 15.000 |
| Carbopol Ultrez 10 polymer | 1.250 |
| Methyl Parabens | 1.000 |
| TiO₂ | 25g (≡ 5%) |

| **Stage C** | |
|---|---|
| Triethanolamine 99% | 1.500 |
| Water pure | 15 |

| **Stage D** | |
|---|---|
| Water pure | 15.000 |
| Melanin | 1.50 (≡ 0.30%) |

| **Stage E** | |
|---|---|
| Water pure | 15.000 |
| Germall 115 | 2.500 |

In this formulation, titanium dioxide (UV scatterer) is used in combination with Parsol 1789 (UV absorber) and our allomelanin. The melanin contributed antioxidant, anti-irritancy and UV-absorbent (UV-A and B) properties as well as imparting an attractive flesh tone to the cream

### B (ii) Melanin-Tinted Moisturiser

| **Material** | **Grams** |
|---|---|
| **Stage A** | |
| Lasemul 92AE | 2.000 |
| Polawax GP200 | 8.000 |
| Grapeseed oil | 4.000 |
| Almond oil | 4.000 |
| Vitamin E acetate | 0.100 |
| Nipasol M | 0.200 |
| Crodamol SS | 3.000 |
| Silicone DC200/350 | 2.000 |
| | |

| **Stage B** | |
|---|---|
| Propylene glycol | 10.000 |
| Nipagin M | 0.200 |
| Titanium Dioxide AHR | 3.000 |
| Yellow iron oxide11556 | 0.200 |
| Red iron oxide 11554 | 0.050 |
| Melanin | 1.000 |
| Water pure | 56.900 |
| | |

| **Stage C** | |
|---|---|
| Biopure 100 | 0.350 |
| Water pure | 5.000 |
| | Batch size 100.000g |

The melanin contributed antioxidant, anti-irritancy and UV-absorbent (UV-A and B) properties as well as imparting an attractive flesh tone to the moisturiser.

### B (iii) ZYLEPSIS MELANIN SHAMPOO

| **Material** | **Quantity** |
|---|---|
| **Stage A** | |
| D-Panthenol USP | 0.200 |
| Nervanaid BA2 | 0.050 |
| Euxyl K400 | 0.200 |
| Water pure | 53.250 |

| **Stage B** | |
|---|---|
| Tegobetaine F50 | 2.000 |
| Surfac LC/X | 25.000 |

| **Stage C** | |
|---|---|
| Melanin | 0.300 |

| **Stage D** | |
|---|---|
| Surfac CDE | 2.000 |

| **Stage E** | |
|---|---|
| Tego Glucoside L55 | 12.000 |

| **Stage F** | |
|---|---|
| Euperlan PK771 | 5.000 |
| | **Batch size 100.000g** |

Here our allomelanin is used in conjunction with a pearlising agent to create an attractive " liquid gold" appearance, without having to use iron salts which are conventionally used to achieve a gold appearance, but which can act as pro-oxidants. In contrast, the melanin serves as an antioxidant, as well as a UV (A and B) absorbent, an anti-irritant agent and a colorant.

Panthenol facilitates penetration of the melanin into the hair (enabled by the solubility of the melanin). This imparts substantivity to the antioxidant and UV absorbent properties and also enables the melanin to tint any grey hair.

### B (iv) HAIR CONDITIONER

| **Stage A** | |
|---|---|
| Dehydquat ACA | 5.000g |
| Stepanquat ML | 2.500g |
| Sodium Hydroxide-5% solution | 1.550g |
| Trivallin SF | 10.000g |
| Nipagin M | 0.200g |
| Water: Pure | 66.100g |
| **Stage B** | |
| Nipasol M | 0.150g |
| Waglinol 6016 | 5.000g |
| Stearyl alcohol | 3.000g |
| Cetyl alcohol | 5.000g |
| **Stage C** | |
| Melanin | 0.500g |
| **Stage D** | |
| Silicone DC 345 | 1.000g |
| Batch Size | 100.000g |

Stages A, B, C and D are mixed to produce the conditioner. Optionally either 0.5g mica/100g cream or 0.20g titanium dioxide/100g cream can be added to provide a pearlescent or whiter appearance

### B (v) Dihydroxyacetone Cream with Melanin

| **Material** | **Grams** |
|---|---|
| **Stage A** | |
| Lexemul 561 | 3.000 |
| Nipasol M | 0.200 |
| Vitamin E Acetate | 0.100 |
| Light Mineral Oil | 5.000 |
| Cetearyl Alcohol | 3.000 |
| Silicone DC 593 | 2.000 |

| **Stage B** | |
|---|---|
| Nipagin M | 0.200 |
| Propylene glycol | 5.000 |
| Nervanaid BA2 | 0.050 |
| Carbopol Ultrez 10 polymer | 0.250 |
| Water, Pure | 60.220 |

| **Stage C** | |
|---|---|
| Triethanolamine 99% | 0.380 |
| Water pure | 5.000 |

| **Stage D** | |
|---|---|
| Melanin | 0.300 |
| Dihydroxyacetone | 5.000 |
| Propylene glycol | 10.000 |
| Biopure 100 | 0.300 |
| | **Batch size 100.000g** |

The allomelanin and dihydroxyacetone cooperate to give an improved skin tan colour, with the melanin also providing antioxidant, anti-irritant and UV absorbing properties.

### B (vi) Self Tanning Lotion with Melanin

| | |
|---|---|
| Aqua | 75.800% |
| Paraffinum Liquidum | 6.500% |
| Octyl Stearate | 5.700% |
| Dihydroxyacetone | 5.000% |
| Glycerin | 3.000% |
| Polyglyceryl-3 Methylglucose Distearate | 2.000% |
| Octyl Stearate | 0.800% |
| Sodium Hydroxide | 0.300% |
| Melanin | 0.250% |
| Carbomer | 0.200% |
| Methyl Paraben | 0.200% |
| Propyl Paraben | 0.100% |
| Parfum | 0.100% |
| Butyl Paraben | 0.050% |
| | |
| | 100.000% |

The melanin gives an improved skin tan colour and provides antioxidant, anti-irritant and UV absorbing properties.

### C Fragrance Stabilisation Test Schedule

To test the stability of a fragrance in the form of an eau de toilette ("EDT"), we use aliquots of the EDT to which are added two or more concentrations of our melanin ("EDT'') and, as reference examples, two concentrations of BHT (butyrated hydroxytoluene, a known antioxidant used in cosmetics). A further aliquot of the original eau de toilette is used as a control. Test samples are made in clear glass bottles that are half filled with the test samples. This is to allow some penetration of UV light and sufficient gas in the headspace to allow oxidative deterioration of the fragrance to occur.

Samples are stored at 37°C overnight, then are cooled to 4°C and then allowed to stand in direct sunlight, for the rest of the day, being returned to 37°C. In addition bottles are opened before and after the 37°C incubation. This daily cycle is repeated for 7 days and the aromas of each assessed.

Our tests have shown our melanin to be markedly superior to BHT, which is markedly superior to the control. The test can also be adapted to show whether a given fragrance composition is markedly susceptible to degradation and whether this is significantly inhibited by our melanin.

### D Anti-Irritancy

### D (i) DHA sensitivity

The effectiveness of our melanin in reducing skin irritation caused by dihydroxyacetone ("DHA") in a subject known to be sensitive to it was investigated.

### Materials

Cream A: Skin cream (oil in water) containing 5.0% DHA.
Cream B: Skin cream (oil in water) containing 0.3% melanin and 5.0% DHA.

### Method

The colour of two areas of skin on the inner lower forearm of the subject was measured in triplicate using the Minolta Chroma Meter. Values were recorded for the degree of lightness, 'L' the degree of redness. 'a' and the degree of yellowness, 'b'. Samples (0.7 gram) of each cream were then applied in random order to the 2 previously measured areas of skin and spread over a circular area of skin 4 cm in diameter. The colour of the skin was re-measured in triplicate 30 and 60 minutes after application of the creams. The mean values of the sets of 3 readings are shown below.

### Results

| Cream A | Colour | | | Change in colour | | |
|---|---|---|---|---|---|---|
| | L | a | b | L | a | b |
| Pre-Test | 67.61 | 7.10 | 12.01 | - | - | - |
| 30 minutes | 63.36 | 10.91 | 11.79 | -4.25 | +3.81 | -0.22 |
| 60 minutes | 64.16 | 10.70 | 14.02 | -3.45 | +3.60 | +2.01 |

| Cream B | Colour | | | Change in colour | | |
|---|---|---|---|---|---|---|
| | L | a | b | L | a | b |
| Pre-Test | 66.34 | 6.54 | 12.35 | - | - | - |
| 30 minutes | 63.29 | 9.70 | 14.00 | -3.05 | +3.16 | +1.65 |
| 60 minutes | 64.66 | 8.97 | 16.23 | -1.68 | +2.43 | +3.88 |

### Conclusion

Both creams caused an increase in redness of the skin (i.e. increase in 'a' value) as well as increasing the darkness and yellow hues.

After 30 minutes, cream A had caused a substantial increase in redness, and a small decrease in yellowness. Thus the skin's appearance was much more red. After 60 minutes the degree of redness had lessened slightly. Yellowness had increased.

Cream B (with melanin) gave rise to a significantly lower value for redness after 30 minutes, and this fell more considerably over the next 30 minutes. Yellowness was higher at 30 minutes (possibly due to the colour of the melanin) and its subsequent increase was very similar to that observed with cream A. It is clear that the melanin has the effect of reducing the initial intensity of irritation (redness), and providing a longer term soothing action.

### D (ii) Surfactant sensitivity

The effectiveness of our melanin in reducing skin irritation due to a surfactant was investigated.

Two human subjects were used. The effect of applying to skin for 24 hours a patch containing a 70% solution of sodium lauryl ether sulphate ('SLES') was compared with the effect of applying an identical patch also containing 0.3% of our melanin ('M'). The colour of the treated skin was monitored for several days using a Chroma Meter. The changes in degrees of redness are tabulated below.

### Subject 1

| Day | 1 | 2 | 6 |
|---|---|---|---|
| Redness Change | +6.3 | +4.2 | +0.3 |
| (SLES) | | | |
| Redness Change | +2.5 | +0.7 | -0.1 |
| (SLES + M) | | | |

### Subject 2

| Day | 1 | 2 | 7 |
|---|---|---|---|
| Redness Change | +3.5 | +2.3 | +0.6 |
| (SLES) | | | |
| Redness Change | +2.5 | +1.4 | -0.2 |
| (SLES + M) | | | |

These results show that a small concentration of our melanin can substantially reduce irritation/inflammation caused by a much larger amount of a surfactant. Clearly the melanin cannot be locking up the surfactant in some way. Probably it is acting at the skin surface, forming a barrier layer and/or having a direct anti-inflammatory effect on skin cells.

The results presented in section D demonstrate anti-inflammatory action against two quite different irritants, and it is therefore reasonable to posit a general anti-inflammatory effect that should reduce irritation due to other causes including sun-induced irritancy. Thus our melanin may be included in sun creams and other cosmetics for a plurality of reasons. Melanin may be applied in a pre-treatment before potential irritants are encountered, and/or in a soothing post-cream for countering irritancy already induced.

## Claims

1. Use of an allomelanin, wherein the allomelanin is produced by enzymatic oxidation of at least one phenol of formula II: 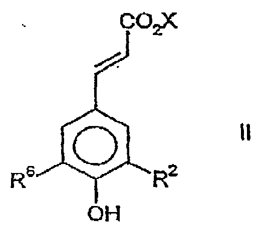 where R² and R⁶ are independently selected from H, OH and OMe and X is H or a C₁₋₆ alkyl group, in the manufacture of a composition for the treatment of inflammation caused by dihydroxyacetone and/or a surfactant.

2. Use of an allomelanin, wherein the allomelanin is produced by enzymatic oxidation of at least one phenol of formula II: 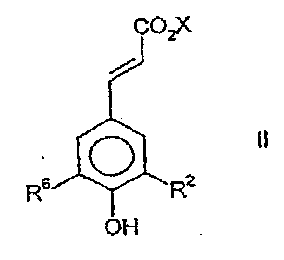 where R² and R⁶ are independently selected from H, OH and OMe and X is H or a C₁₋₆ alkyl group, in the manufacture of a composition for the treatment of irritancy caused by dihydroxyacetone and/or a surfactant.

3. Use of an allomelanin as claimed in claim 1 or 2, wherein the dihydroxyacetone and/or surfactant is present in the composition.

4. Use of an allomelanin as claimed in any of claims 1 to 3, wherein the dihydroxyacetone and/or surfactant is present in other compositions that may be applied to the skin.

5. Use of an allomelanin, according to Claim 1 or Claim 2, wherein the composition is a cosmetic composition

6. Use of an allomelanin as claimed in Claim 5, wherein the dihydroxyacetone and/or surfactant is present in the composition.

7. Use of an allomelanin as claimed in claim 5 or 6, wherein the dihydroxyacetone and/or surfactant is present in other compositions that may be applied to the skin.

8. A use according to any preceding claim, wherein the allomelanin has a water solubility of at least 50 g/l at room temperature.

9. A use according to any of claims 5 to 7, wherein the cosmetic composition is a self-tanning composition.

10. A use according to any of claims 5 to 7, wherein the cosmetic composition contains 0.4-10% by weight of dihydroxyacetone.

11. A use according to any of claims 5 to 7, wherein the cosmetic composition contains 2-5% by weight of dihydroxyacetone

12. A use according to any preceding claim, wherein the composition contains 0.05-0.5% by weight of said allomelanin.

13. A use according to any preceding claim, wherein the composition contains 0.1-0.3% by weight of said allomelanin.

14. A use according to any preceding claim, wherein the allomelanin is produced by enzymatic oxidation of caffeic acid and/or ferulic acid.

15. A use according to any preceding claim wherein the enzyme is selected from peroxidases, tyrosinases and laccases.

16. A use according to any preceding claim, wherein the composition is a topical composition.

## Patentansprüche

1. Verwendung eines Allomelanins, wobei das Allomelanin mittels enzymatischer Oxidation von mindestens einem Phenol der Formel II: 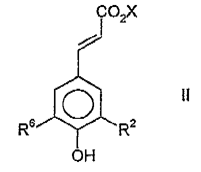 hergestellt wird,
wobei R² und R⁶ unabhängig ausgewählt werden aus H, OH und OMe, und X H oder eine C₁₋₆-Alkylgruppe ist, zur Herstellung einer Zusammensetzung für die Behandlung einer durch Dihydroxyazeton und/oder durch eine oberflächenaktive Substanz verursachten Entzündung.

2. Verwendung eines Allomelanins, wobei das Allomelanin mittels enzymatischer Oxidation von mindestens einem Phenol der Formel II: 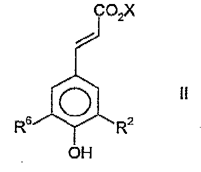 hergestellt wird,
wobei R² und R⁶ unabhängig ausgewählt werden aus H, OH und OMe, und X H oder eine C₁₋₆-Alkylgruppe ist, zur Herstellung einer Zusammensetzung für die Behandlung einer durch Dihydroxyazeton und/oder durch eine oberflächenaktive Substanz verursachten Reizung.

3. Verwendung eines Allomelanins wie in Anspruch 1 oder 2 beansprucht, wobei das Dihydroxyazeton und/oder die oberflächenaktive Substanz in der Zusammensetzung vorhanden sind.

4. Verwendung eines Allomelanins wie in irgendeinem der Ansprüche 1 bis 3 beansprucht, wobei das Dihydroxyazeton und/oder die oberflächenaktive Substanz in anderen Zusammensetzungen, die auf die Haut aufgetragen werden können, vorhanden sind.

5. Verwendung eines Allomelanins nach Anspruch 1 oder Anspruch 2, wobei die Zusammensetzung eine kosmetische Zusammensetzung ist.

6. Verwendung eines Allomelanins wie in Anspruch 5 beansprucht, wobei das Dihydroxyazeton und/oder die oberflächenaktive Substanz in der Zusammensetzung vorhanden sind.

7. Verwendung eines Allomelanins wie in Anspruch 5 oder 6 beansprucht, wobei das Dihydroxyazeton und/oder die oberflächenaktive Substanz in anderen Zusammensetzungen, die auf die Haut aufgetragen werden können, vorhanden sind.

8. Verwendung nach irgendeinem der vorhergehenden Ansprüche, wobei das Allomelanin eine Wasserlöslichkeit von mindestens 50g/l bei Raumtemperatur hat.

9. Verwendung nach irgendeinem der Ansprüche 5 bis 7, wobei die kosmetische Zusammensetzung eine Selbstbräunungszusammensetzung ist.

10. Verwendung nach irgendeinem der Ansprüche 5 bis 7, wobei die kosmetische Zusammensetzung 0,4-10 Gew.-% Dihydroxyazeton enthält.

11. Verwendung nach irgendeinem der Ansprüche 5 bis 7, wobei die kosmetische Zusammensetzung 2-5 Gew.-% Dihydroxyazeton enthält.

12. Verwendung nach irgendeinem der vorhergehenden Ansprüche, wobei die Zusammensetzung 0,05-0,5 Gew.-% des Allomelanins enthält.

13. Verwendung nach irgendeinem der vorhergehenden Ansprüche, wobei die Zusammensetzung 0,1-0,3 Gew.-% des Allomelanins enthält.

14. Verwendung nach irgendeinem der vorhergehenden Ansprüche, wobei das Allomelanin mittels enzymatischer Oxidation von Kaffeesäure und/oder Ferulasäure hergestellt wird.

15. Verwendung nach irgendeinem der vorhergehenden Ansprüche, wobei das Enzym ausgewählt wird aus Peroxidasen, Tyrosinasen und Laccasen.

16. Verwendung nach irgendeinem der vorhergehenden Ansprüche, wobei die Zusammensetzung eine topische Zusammensetzung ist.

## Revendications

1. Utilisation d'une allomélanine, dans laquelle l'allomélanine est produite par une oxydation enzymatique d'au moins un phénol de la formule II: 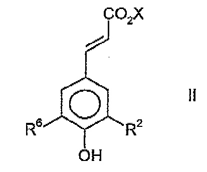 où R² et R⁶ sont indépendamment choisis parmi H, OH et OMe et X est H ou un groupe alkyle C₁₋₆, dans la fabrication d'une composition pour le traitement d'une inflammation entraînée par la dihydroxyacétone et/ou un tensioactif.

2. Utilisation d'une allomélanine, dans laquelle l'allomélanine est produite par une oxydation enzymatique d'au moins un phénol de la formule II: 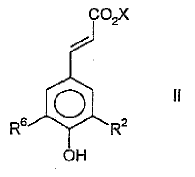 où R² et R⁶ sont indépendamment choisis parmi H, OH et OMe et X est H ou un groupe alkyle C₁₋₆, dans la fabrication d'une composition pour le traitement d'une irritation entraînée par la dihydroxyacétone et/ou un tensioactif.

3. Utilisation d'une allomélanine suivant la revendication 1 ou 2, dans laquelle la dihydroxyacétone et/ou le tensioactif sont présents dans la composition.

4. Utilisation d'une allomélanine suivant l'une quelconque des revendications 1 à 3, dans laquelle la dihydroxyacétone et/ou le tensioactif sont présents dans d'autres compositions qui peuvent être appliquées sur la peau.

5. Utilisation d'une allomélanine suivant la revendication 1 ou 2, dans laquelle la composition est une composition cosmétique.

6. Utilisation d'une allomélanine suivant la revendication 5, dans laquelle la dihydroxyacétone et/ou le tensioactif sont présents dans la composition.

7. Utilisation d'une allomélanine suivant la revendications 5 ou 6, dans laquelle la dihydroxyacétone et/ou le tensioactif sont présents dans d'autres compositions qui peuvent être appliquées sur la peau.

8. Utilisation suivant l'une quelconque des revendications précédentes, dans laquelle l'allomélanine possède une solubilité dans l'eau d'au moins 50 g/l à la température ambiante.

9. Utilisation suivant l'une quelconque des revendications 5 à 7, dans laquelle la composition cosmétique est une composition auto-bronzante,

10. Utilisation suivant l'une quelconque des revendications 5 à 7, dans laquelle la composition cosmétique contient 0,4-10% en poids de dihydroxyacétone.

11. Utilisation suivant l'une quelconque des revendications 5 à 7, dans laquelle la composition cosmétique contient 2-5% en poids de dihydroxyacétone.

12. Utilisation suivant l'une quelconque des revendications précédentes, dans laquelle la composition contient 0,05-0,5% en poids de ladite allomélanine.

13. Utilisation suivant l'une quelconque des revendications précédentes, dans laquelle la composition contient 0,1-0,3% en poids de ladite allomélanine.

14. Utilisation suivant l'une quelconque des revendications précédentes, dans laquelle l'allomélanine est produite par une oxydation enzymatique d'acide caféique et/ou d'acide férulique.

15. Utilisation suivant l'une quelconque des revendications précédentes, dans laquelle l'enzyme est choisie parmi des peroxydases, des tyrosinases et des laccases.

16. Utilisation suivant l'une quelconque des revendications précédentes, dans laquelle la composition est une composition topique.
